(19) Europäisches Patentamt / European Patent Office / Office européen des brevets

(11) **EP 2 956 446 B1**

(12) **FASCICULE DE BREVET EUROPEEN**

(45) Date de publication et mention de la délivrance du brevet:
**17.01.2018 Bulletin 2018/03**

(21) Numéro de dépôt: **14706900.9**

(22) Date de dépôt: **06.02.2014**

(51) Int Cl.:
***C07D 301/03*** *(2006.01)*

(86) Numéro de dépôt international:
**PCT/FR2014/050226**

(87) Numéro de publication internationale:
**WO 2014/125191 (21.08.2014 Gazette 2014/34)**

(54) **PROCÉDÉ DE PRODUCTION D'OXYDE D'ÉTHYLÈNE A PARTIR D'UN FLUX D'ÉTHANOL THERMO-MÉCANIQUEMENT INTÉGRÉ**

VERFAHREN ZUR HERSTELLUNG VON ETHYLENOXID AUS EINEM THERMOMECHANISCH INTEGRIERTEN ETHANOLSTROM

METHOD FOR PRODUCING ETHYLENE OXIDE FROM A THERMO-MECHANICALLY INTEGRATED ETHANOL STREAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.02.2013 FR 1351161**

(43) Date de publication de la demande:
**23.12.2015 Bulletin 2015/52**

(73) Titulaire: **IFP Energies nouvelles
92500 Rueil-Malmaison (FR)**

(72) Inventeurs:
• **COUPARD, Vincent
F-69100 Villeurbanne (FR)**
• **PLENNEVAUX, Thomas
F-69003 Lyon (FR)**

(56) Documents cités:
**WO-A2-2007/134415    US-A- 3 119 837
US-A- 4 396 789**

• **BRUSCINO M: "Scientific Design's Ethanol to Monoethylene Glycol Technology", HIDROCARBON WORLD,, vol. 5, no. 2, 1 janvier 2010 (2010-01-01), pages 12, 14-16, XP009171109, ISSN: 1753-3899 cité dans la demande**

EP 2 956 446 B1

**Description**

**Domaine de l'invention**

La présente invention se rapporte à un procédé de transformation de l'éthanol en oxyde d'éthylène incluant une étape de déshydration de l'éthanol et une étape d'oxydation de l'éthylène produit intégrées.

**État de la technique antérieure**

La réaction de déshydratation de l'éthanol en éthylène est connue et détaillée depuis la fin du XIXème siècle. "The Deshydration of Alcohols over Alumina. I:The reaction scheme", H. Knözinger, R. Köhne, Journal of Catalysis (1966), 5, 264-270 est considérée comme la publication de base sur les travaux de déshydratation des alcools dont l'éthanol. Il est connu que cette réaction est très endothermique, équilibrée et déplacée vers l'éthylène à haute température. La chute de température correspondant à la conversion totale de l'éthanol pur dans un réacteur adiabatique est de 380°C. À plus basse température, l'éthanol est converti en diéthyl éther (DEE). Cet "intermédiaire" de réaction peut être présent dans des procédés de déshydratation de l'éthylène dans lesquelles la conversion est partielle ou entre deux réacteurs dans des procédés multi-réacteurs. Le DEE peut ensuite être converti en éthylène à plus haute température. Le catalyseur de référence souvent utilisé est un catalyseur monofonctionnel acide, l'alumine gamma étant le catalyseur le plus cité. Les zéolithes sont également utilisées pour cette application, en particulier la ZSM5 depuis les années 1980, comme par exemple dans "Reactions of ethanol over ZSM-5",S.N. Chaudhuri & al., Journal of Molecular Catalysis 62:289-295 (1990).

Le brevet US 4,232,179 décrit un procédé de déshydratation de l'éthanol en éthylène dans lequel la chaleur nécessaire à la réaction est apportée par l'introduction dans le réacteur d'un fluide caloporteur en mélange avec la charge. Le fluide caloporteur est soit de la vapeur d'eau provenant d'une source externe, soit un flux externe provenant du procédé, soit le recycle d'une partie de l'effluent du réacteur de déshydration, c'est à dire l'éthylène produit. L'introduction du mélange de la charge avec ledit fluide caloporteur permet de fournir la chaleur nécessaire au maintien de la température du lit catalytique à un niveau compatible avec les conversions désirées. Dans le cas où le fluide caloporteur est l'effluent du réacteur de déshydratation, un compresseur de recyclage dudit effluent est nécessaire. Cependant, le recyclage de l'éthylène produit par la réaction est un inconvénient car l'introduction de l'éthylène modifie l'équilibre de la réaction de déshydratation. De plus, l'éthylène participe aux réactions secondaires d'oligomérisation, de transfert d'hydrogène et de disproportionation des oléfines qui sont des réactions d'ordre supérieur à 0 par rapport à leur réactif. L'augmentation de la concentration en éthylène dès le début de la réaction multiplie la formation de produits secondaires. La perte en éthylène est donc plus importante, ce qui se traduit par une baisse de la sélectivité.

La demande de brevet WO 2007/134415 décrit un procédé de déshydratation de l'éthanol en éthylène amélioré par rapport à celui du brevet US 4 232 179 permettant un coût d'investissement réduit, grâce à un nombre réduit d'équipements et un coût opérationnel réduit, grâce à la non utilisation de vapeur d'eau externe au procédé. Dans ce procédé, au moins une partie de l'effluent du réacteur de déshydratation (mélange d'éthylène produit et de vapeur d'eau) et de la vapeur d'eau surchauffée obtenue à partir de l'eau produite par la déshydratation de l'éthanol et condensée dans le réacteur, sont utilisés comme fluide caloporteur et entrent dans le réacteur de déshydratation en mélange avec l'éthanol. Ladite demande de brevet est muette sur la condition de pression à respecter entre la charge éthanol et l'effluent dans le but de maximiser l'échange de chaleur.

Le brevet US 4,396,789 décrit également un procédé de déshydratation de l'éthanol en éthylène dans lequel l'éthanol et la vapeur d'eau agissant comme fluide caloporteur sont introduits dans le premier réacteur à une température comprise entre 400 et 520°C et à une pression élevée comprise entre 20 et 40 atm, de sorte que l'effluent produit par la réaction de déshydratation est soutiré du dernier réacteur à une pression au moins supérieure à 18 atm, ledit produit de réaction, c'est à dire l'éthylène, pouvant subir après refroidissement l'étape de distillation cryogénique finale sans étape de compression intermédiaire. Ledit procédé se caractérise également par un échange de chaleur entre ledit produit de la réaction de déshydratation et la charge introduite dans le premier réacteur, ledit produit de réaction étant utilisé pour vaporiser la charge entrant dans le premier réacteur. L'éthanol non converti, au moins une partie de l'eau formée au cours des réactions du procédé et l'eau ajoutée pour le lavage final des gaz sont recyclés pour assurer la conversion complète de l'éthanol.

La demande de brevet WO 2011/002699 divulgue un procédé de déshydratation d'une charge éthanol en éthylène comprenant la vaporisation d'un mélange d'éthanol et d'eau et la réaction de ce mélange dans un réacteur adiabatique. Cette demande n'adresse pas le problème de la maximisation de la récupération de chaleur en vue de réduire la consommation énergétique du procédé.

Le brevet US3119837 décrit un procédé d'oxydation d'éthylène en présence d'un catalyseur a base d'argent. Il s'appuie sur l'effet positif du méthane sur la sélectivité de la réaction de conversion de l'éthylène en oxyde d'éthylène et donne les conditions opératoires de l'oxydation en présence d'oxygène purifié. Les principales conditions opératoires données sont les suivantes : température comprise entre 200°C et 300°C (préférentiellement)et pression comprise entre 15 et 500 psi abs (préférentiellement).

Le brevet EP0496470B1 décrit un catalyseur d'oxydation pouvant être utilisé dans le procédé décrit par le brevet US3119837. Ce brevet décrit les principale caractéristiques du catalyseur ainsi que les gammes de conditions opératoires (température) dans lesquelles il peut être utilisé. Le catalyseur décrit est a base d'argent et les conditions opératoires sont compatibles avec celles décrite dans le brevet US3119837.

Mike Bruscino, "Scientific Design's Ethanol to Monoethylene Glycol Technology", Hydrocarbon World, Vol.5(2), pp.15-17 (2010) décrit un procédé de production d'éthylène glycol à partir d'éthanol biosourcé incluant un procédé de déshydratation d'éthanol en éthylène, un procédé d'oxydation de l'éthylène ainsi produit et un procédé de conversion de l'oxyde d'éthylène en glycol. Le procédé décrit ne considère par de recyclage de l'eau vers la section de déshydratation ni d'intégration thermique entre la section d'oxydation et la section de déshydratation.

Un objectif de l'invention est de fournir un procédé de production d'oxyde d'éthylène comprenant une étape de déshydratation de l'éthanol en éthylène et une étape d'oxydation catalytique de l'éthylène produit, ces deux étapes étant intégrées l'une a l'autre afin de limiter la consommation énergétique et la consommation d'eau.

## Résumé et intérêt de l'invention

L'invention décrit un procédé de production d'oxyde d'éthylène a partir d'éthanol. Ce procédé consiste en l'intégration de deux procédés distincts : la déshydratation catalytique de l'éthanol, et l'oxydation de l'éthylène produit.

Le procédé de déshydratation d'une charge éthanol en éthylène comprenant une étape de prétraitement qui réduit le taux d'azote organique ou basique contenu dans ladite charge et convertit une fraction de l'éthanol en DEE, et une étape de vaporisation de la charge éthanol prétraitée, en mélange avec au moins une partie d'un flux d'eau de dilution comprenant de l'éthanol recyclés, dans un échangeur grâce à un échange de chaleur avec l'effluent issu du dernier réacteur de déshydratation. Le procédé d'oxydation catalytique met en oeuvre au moins un réacteur multitubulaire refroidi par vaporisation d'un fluide caloporteur dans la calandre du réacteur, ledit fluide comprenant préférentiellement de l'eau. L'énergie thermique extraite du réacteur via le fluide caloporteur vaporisé est utilisée comme énergie mécanique pour comprimer la charge de déshydratation et permettre l'intégration thermique charge/effluent de la section de déshydratation.

Ladite invention présente l'avantage par rapport aux procédés de l'art antérieur de réduire la consommation d'eau et de diminuer la consommation énergétique nécessaire pour convertir l'éthanol en oxyde d'éthylène.

En particulier, la présente invention permet de diminuer la consommation d'énergie mécanique nécessaire à la compression, ce qui est particulièrement avantageux, notamment quand les besoins thermiques sont couverts par ailleurs.

L'oxyde d'éthylène produit est un intermédiaire réactionnel dans la synthèse de nombreux produits, par exemple les glycols, les polyols, d'éthoxylates, éthers de glycol, éthanol-amines.

## Description de l'invention

L'invention concerne un procédé de déshydratation d'une charge éthanol en éthylène puis oxydation de l'éthylène en oxyde d'éthylène comprenant :

a) éventuellement une étape de préchauffe de ladite charge éthanol à une température comprise entre 70 et 130°C par échange de chaleur avec refluent issu de l'étape e),
b) une étape de prétraitement de la charge éthanol sur un solide acide opérant à une température comprise entre 70 et 130°C de manière à produire une charge éthanol prétraitée,
c) Une étape de vaporisation d'une charge de vaporisation comprenant ladite charge éthanol prétraitée et au moins une partie du flux d'eau de dilution comprenant de l'éthanol recyclé à l'issue de l'étape g) dans un échangeur grâce à un échange de chaleur avec l'effluent issu du dernier réacteur de l'étape e), ladite charge de vaporisation étant introduite dans ladite étape de vaporisation à une pression comprise entre 0,1 et 1,4 MPa, choisie la plus haute possible, de manière à ce que la différence entre la température de condensation de l'effluent de l'étape e) de déshydratation et la température de vaporisation de ladite charge de vaporisation soit supérieure ou égale à 2 °C. de manière à produire une charge vaporisée,
d) Une étape de compression et surchauffe de ladite charge vaporisée dans un compresseur de manière à produire une charge comprimée et à la température d'alimentation de la section de déshydratation e), ledit compresseur étant entraîné par une turbine à condensation prenant à l'aspiration le flux de trempe vaporisé dans la section i) d'oxydation, le flux de décharge sortant de la turbine à condensation étant recyclé vers l'étape i) d'oxydation,
e) Une étape de déshydratation de la charge comprimée, ladite charge présentant un rapport massique eau sur éthanol compris entre 1 et 4, dans au moins un réacteur adiabatique contenant au moins un catalyseur de déshydratation et dans lequel la réaction de déshydratation a lieu, opérant à une température d'entrée comprise entre 350 et 550 °C et à une pression d'entrée comprise entre 0,3 et 1,8 MPa,

f) Une étape de séparation de l'effluent issu du dernier réacteur adiabatique de l'étape e) en un effluent comprenant de l'éthylène à une pression inférieure à 1,6 MPa et un effluent comprenant de l'eau,

g) Une étape de purification d'au moins une partie de l'effluent comprenant de l'eau issu de l'étape f) et la séparation en au moins un flux d'eau traitée et un flux d'eau de dilution comprenant de l'éthanol, ce dernier étant recyclé en amont de l'étape c) de vaporisation,

h) Une étape de compression de l'effluent comprenant de l'éthylène issu de l'étape f),

i) Une étape d'oxydation de l'effluent issu de l'étape h) en oxyde d'éthylène, cette étape d'oxydation comprenant au moins un réacteur tubulaire d'oxydation refroidi par vaporisation dudit flux de trempe issu de l'étape d), ledit flux trempe ainsi réchauffé étant recyclé vers l'étape d).

**Charge**

Conformément à l'invention, la charge traitée dans le procédé est une charge éthanol.

Ladite charge éthanol est avantageusement une charge éthanol concentrée. On entend par charge éthanol concentrée une charge éthanol comprenant un pourcentage massique en éthanol supérieur ou égal à 35% poids. De préférence, ladite charge éthanol concentrée comprend un pourcentage massique en éthanol compris entre 35 et 99,9% poids.

La charge éthanol comprenant moins de 35% poids d'éthanol peut être concentrée par tous moyens connus de l'Homme du métier, par exemple par distillation, par absorption, par pervaporation.

Ladite charge éthanol comprend également avantageusement, en plus de l'eau, une teneur en alcools autres que l'éthanol, tels que par exemple le méthanol, le butanol et/ou l'isopentanol inférieure à 10 % poids, et de préférence inférieure à 5 % poids, une teneur en composés oxygénés autres que les alcools tels que par exemple les éthers, les acides, les cétones, les aldéhydes et/ou les esters inférieure à 1 % poids et une teneur en azote et en soufre, organique et minéral, inférieure à 0,5 % poids, les pourcentages poids étant exprimés par rapport à la masse totale de ladite charge.

La charge éthanol traitée dans le procédé selon l'invention est éventuellement obtenue par un procédé de synthèse d'alcool à partir de ressources fossiles telles que par exemple à partir du charbon, du gaz naturel ou des déchets carbonés.

Ladite charge peut également avantageusement provenir de ressources non fossiles. De préférence, la charge éthanol traitée dans le procédé selon l'invention est une charge éthanol produite à partir de source renouvelable issue de la biomasse, souvent appelée "bioéthanol". Le bioéthanol est une charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau.

Pour une description plus complète des procédés fermentaires classiques, on peut se reporter a l'ouvrage 'Les Biocarburants, État des lieux, perspectives et enjeux du développement, Daniel Ballerini, Editions Technip'.

Ladite charge peut également avantageusement être obtenue à partir de gaz de synthèse.

Ladite charge peut également avantageusement également être obtenue par hydrogénation des acides ou esters correspondants. Dans ce cas, l'acide acétique ou les esters acétiques sont avantageusement hydrogénés à l'aide d'hydrogène en éthanol. L'acide acétique peut avantageusement être obtenu par carbonylation du méthanol ou par fermentation des carbohydrates.

De préférence, la charge éthanol traitée dans le procédé selon l'invention est une charge éthanol produite à partir de source renouvelable issue de la biomasse.

**Étape a) de préchauffe**

La charge éthanol subit éventuellement une étape a) de préchauffe dans un échangeur de chaleur de manière à produire une charge éthanol préchauffée, grâce à un échange de chaleur avec l'effluent issu de l'étape e) de déshydratation. Lorsqu'une étape b) de prétraitement est mise en oeuvre, la température à l'issue de l'étape a) est comprise entre 70 et 130 °C, préférentiellement entre 110°C et 130°C. La pression de la charge éthanol est ajustée de telle manière que celle ci reste liquide à l'issue de l'étape a) de préchauffe, à une valeur comprise entre 0,1 et 3 MPa.

**Étape b) de prétraitement**

La charge éthanol, éventuellement préchauffée subit une étape b) de prétraitement de manière à produire une charge éthanol prétraitée. Ladite étape de prétraitement permet d'éliminer les composés azotés présents dans ladite charge préchauffée de manière à limiter la désactivation du catalyseur de déshydratation placé en aval.

Ladite étape b) de prétraitement est mise en oeuvre sur un solide acide, de préférence une résine acide, et à une température comprise entre 70 et 130 °C, préférentiellement entre 110°C et 130°C.

Ladite étape b) de prétraitement permet d'éliminer les impuretés, basiques et/ou organiques, et les espèces cationiques afin d'obtenir une charge éthanol prétraitée répondant au niveau d'impuretés compatibles avec le catalyseur de déshydratation.

Le prétraitement sur le solide acide dans les conditions opératoires selon l'invention permet de convertir entre 3% poids et 20% poids, préférentiellement entre 8 et 12% poids de l'éthanol présent dans ladite charge en DEE, le pourcentage poids étant déterminé par rapport au poids total d'éthanol présent dans ladite charge en entrée de l'étape b) de prétraitement.

Le solide acide comprend tout les solides acides connus par l'Homme de l'art : des silice-alumines, des argiles acides, des zéolithes, des zircones sulfatées, des résines acides, etc. L'essentiel est que le solide acide possède une capacité d'échange élevée pour capter le plus possible les espèces basiques et cationiques et une force d'acidité suffisamment élevée pour effectuer la transformation partielle de l'éthanol en DEE.

Des solides acides qui sont communément disponibles commercialement sont les argiles traitées aux acides pour les rendre acides (comme la montmorillonite) et les zéolithes, ayant un rapport silice sur alumine dans le réseau cristallin de 2.5 à 100 molaire.

La résine acide comprend des groupes sulfoniques, greffées sur un support organique composé de chaines aromatiques et/ou haloaliphatiques. De préférence les solides acides possèdent une capacité d'échange d'au moins de 0,1 mmol $H^+$ équivalent par gramme.

La résine acide est préparée par polymérisation ou co-polymérisation de groupe vinyliques aromatiques suivie d'une sulfonation, lesdits groupes vinyliques aromatiques étant choisis parmi le styrène, le vinyle toluène, le vinyle naphtalène, le vinyle éthyle benzène, le méthyle styrène, le vinyle chlorobenzene et le vinyle xylène, ladite résine présentant un taux de réticulation compris entre 20 et 35 %, de préférence entre 25 et 35 % et de manière préférée, égale à 30 % et une force acide dosée par potentiométrie lors de la neutralisation par une solution de KOH, de 0,2 à 10 mmol $H^+$ équivalent par gramme et de préférence entre 0,2 et 2,5 mmol $H^+$ équivalent par gramme.

Ladite résine échangeuse d'ions acide contient entre 1 et 2 groupes sulfoniques terminaux par groupe aromatique. Sa taille est comprise entre 0,15 et 1,5 mm. Par taille de la résine on entend le diamètre de la plus petite sphère englobant la particule de résine. On mesure les classes de taille de résine par tamisages sur des tamis adaptés selon une technique connue de l'Homme du métier.

Une résine préférée est une résine constituée par des co-polymères de monovinyle aromatiques et polyvinyle aromatiques, et de manière très préférée, de copolymère de di-vinyle benzène et de polystyrène présentant un taux de réticulation compris entre 20 et 45 %, de préférence entre 30 et 40 %, et de manière préférée égale à 35 % et une force acide, représentant le nombre de sites actifs de ladite résine, dosée par potentiométrie lors de la neutralisation par une solution de KOH, compris entre 1 et 10 mmol $H^+$ équivalent par gramme et de préférence compris entre 3,5 et 6 mmol $H^+$ équivalent par gramme. Par exemple, la résine est une résine TA801 vendue par société Axens.

Les solides acides peuvent être régénérés de temps en temps une fois que la capacité d'échange est presque saturée par l'adsorption des espèces basiques et cationiques in situ ou ex situ. Dans le cas des solides acides inorganiques comme les argiles et zéolithes, la régénération peut consister en un simple chauffage à haute température afin de désorber les espèces basiques en présence d'un flux inerte ou contenant de l'oxygène. Les cations peuvent être enlevés par échange ionique. Les résines acides peuvent être régénérées par échange ionique, typiquement par un traitement avec un acide en phase liquide. Les solides acides peuvent également être utilisés une fois jusqu'à saturation et remplacés par du solide vierge.

Le solide acide peut être utilisé seul ou en mélange avec d'autres types de solides acide. Des mélanges de différents solides acides ou des séquences de solides acides peuvent être mises en oeuvre afin d'optimiser la capacité d'adsorber les espèces basiques et cationiques et la capacité de transformer partiellement l'éthanol en DEE.

Le prétraitement décrit ci-dessus peut avantageusement être complété par un prétraitement utilisant une résine échangeuse d'anion. Cette résine peut par exemple être une résine chargée en sodium, ou trimethylamonium caractérisée par une capacité d'échange mesurée en mg($OH^-$/litre). Cette résine peut par exemple être de la résine Amberlite IRN78. Cette résine supplémentaire permet se retenir les ions sulfates $SO_4^{2-}$ afin de prolonger la vie du catalyseur.

**Étape c) de vaporisation**

On appelle charge de vaporisation le mélange comprenant ladite charge éthanol prétraitée et au moins une partie du flux d'eau de dilution comprenant de l'éthanol recyclé à l'issue de l'étape g) de purification.

Conformément à l'invention, le procédé de déshydratation comprend une étape c) de vaporisation de ladite charge de vaporisation de manière à produire une charge vaporisée. Ladite vaporisation est réalisée grâce à un échange de chaleur avec l'effluent issu de l'étape e) de déshydratation dans un échangeur de chaleur.

De préférence, ladite charge de vaporisation est introduite dans ladite étape c) de vaporisation à une pression inférieure à la pression de l'effluent issu de l'étape e) de déshydratation.

La pression de ladite charge de vaporisation en amont de l'étape c) de vaporisation est avantageusement choisie de façon à ce que l'écart de température dans l'échangeur de chaleur entre l'effluent issu de l'étape e) de déshydratation qui se condense et ladite charge de vaporisation qui s'évapore est au moins supérieur à 2°C, et de préférence au moins supérieur à 3°C.

On appelle approche thermique cet écart de température dans l'échangeur de chaleur.

L'ajustement de ladite pression en amont de l'étape c) de vaporisation est un critère essentiel de la présente invention. Cette pression est choisie la plus haute possible, de manière à ce que la différence entre la température de condensation de l'effluent de l'étape e) de déshydratation et la température de vaporisation de ladite charge de vaporisation soit supérieure ou égale à 2 °C, de préférence supérieure ou égale à 3 °C, de manière à maximiser l'échange de chaleur entre ladite charge de vaporisation et ledit effluent issu de l'étape e) de déshydratation.

Dans un arrangement préféré où l'étape b) de prétraitement est réalisée, la température de vaporisation de la charge de vaporisation à pression donnée est abaissée par rapport à celle d'une charge obtenue par un enchaînement qui ne comprendrait pas l'étape b) de prétraitement. Pour une température de condensation de l'effluent de l'étape e) de déshydratation donnée et une approche thermique fixée, on peut donc ajuster la pression en amont de l'étape c) de vaporisation à une valeur plus élevée que ce qu'elle aurait été dans un enchaînement ne comprenant pas l'étape b) de prétraitement.

L'ajustement de ladite pression en amont de l'étape c) de vaporisation à la valeur la plus haute possible, dans les limites déterminées au paragraphe précédent, permet de minimiser l'énergie nécessaire à la compression lors de l'étape d) de compression du procédé selon l'invention.

Ladite charge de vaporisation est introduite dans ladite étape de c) de vaporisation à une pression comprise entre 0,1 et 1,4 MPa, préférentiellement entre 0,2 et 0,6 MPa.

L'introduction de ladite charge de vaporisation dans l'étape c) de vaporisation à ce niveau de pression spécifique compris entre 0,1 et 1,4 MPa, préférentiellement entre 0,2 et 0,6 MPa, inférieure à la pression de l'effluent en sortie du dernier réacteur de l'étape e) de déshydratation, permet de bénéficier d'une température de vaporisation de ladite charge de vaporisation inférieure à la température de condensation de l'effluent issu du dernier réacteur adiabatique. Ainsi, la majeure partie de la chaleur latente de la phase aqueuse de l'effluent issu du dernier réacteur adiabatique est récupérée pour vaporiser ladite charge de vaporisation, sans apport de chaleur externe. La totalité de l'enthalpie de vaporisation de ladite charge de vaporisation est donc échangée avec l'enthalpie de condensation dudit effluent.

**Étape d) de compression et surchauffe**

Selon l'invention, ladite charge vaporisée, subit une compression dans une étape d) de compression de manière à produire une charge comprimée. Ladite étape d) de compression est avantageusement mise en oeuvre dans tout type de compresseur connu de l'Homme du métier. En particulier, l'étape d) de compression est avantageusement mise en oeuvre dans un compresseur de type compresseur radial à multiplicateur intégré ou dans un compresseur comprenant une ou plusieurs soufflantes avec une roue radiale mise en série sans refroidissement intermédiaire ou dans un compresseur de type volumétrique avec ou sans lubrification.

La puissance mécanique nécessaire a la compression est apportée par une turbine à condensation, prenant à l'aspiration le flux de trempe (12) vaporisé dans la section i) d'oxydation. Le flux de décharge (13) sortant de la turbine à condensation est recyclé vers l'étape i) d'oxydation pour y être de nouveau vaporisé grâce a un échange de chaleur avec le milieu réactionnel de l'étape i). Optionnellement, afin de palier à des régimes transitoires, cette puissance peut être complétée par la présence d'une machine tournante d'un type connu de l'homme du métier, par exemple un moteur électrique ou une seconde turbine a vapeur ou a gaz sur l'arbre moteur du compresseur de l'étape d).

Dans un arrangement où l'étape b) est réalisée, ladite étape b) permettant d'opérer à plus haute pression en amont de l'étape c), le taux de compression nécessaire dans l'étape d) est réduit pour atteindre une pression donnée à l'issue de ladite étape d), réduisant ainsi la consommation énergétique de ladite étape d).

L'étape d) de compression permet de réaliser une pompe à chaleur intégrée audit procédé, utilisant les flux issus du procédé, et ne faisant pas intervenir de fluide caloporteur externe.

La combinaison des conditions opératoires spécifiques de l'étape c) et de l'étape d) permet d'éviter l'apport de fluide caloporteur externe au procédé pour assurer la vaporisation de ladite charge de vaporisation en récupérant la majeure partie de la chaleur latente de la phase aqueuse de l'effluent issu du dernier réacteur adiabatique pour vaporiser la charge de vaporisation. Ainsi, seuls les flux issus du procédé sont utilisés

La pression de ladite charge comprimée à l'issue de l'étape d) de compression est avantageusement comprise entre 0,3 et 1,8 MPa, préférentiellement entre 0,5 et 1,3 MPa. La pression de sortie de ladite charge est suffisamment élevée pour que la température de condensation de l'effluent issus du dernier réacteur soit supérieure à la température de vaporisation de la charge entrant dans l'étape c), ce qui est une condition nécessaire à la faisabilité de l'étape c).

Ladite charge comprimée issue de l'étape d) de compression est éventuellement chauffée dans un échangeur de type monophasique gaz, grâce à un échange de chaleur avec l'effluent issu du dernier réacteur adiabatique de l'étape e). Dans ledit échangeur de type monophasique gaz, ladite charge comprimée, est surchauffée et l'effluent issu, à l'état gazeux, du dernier réacteur adiabatique de l'étape e) est "désurchauffé" sans être condensé.

Ladite charge comprimée est avantageusement surchauffée à une température de sortie comprise entre 250 et 420 °C et de préférence comprise entre280 et 410°C. À l'issue dudit échangeur de type monophasique gaz, l'effluent issu, à l'état gazeux, du dernier réacteur adiabatique de l'étape e) présente avantageusement une température comprise entre 180 et 260 °C.

Ainsi, l'utilisation des différents échangeurs, de type monophasique gaz et évaporateur gaz/liquide, et la vaporisation, à une pression inférieure à la pression de l'effluent en sortie du dernier réacteur, de ladite charge de vaporisation, permet la condensation d'au moins 80% des vapeurs d'eau présentes dans l'effluent issu du dernier réacteur de l'étape e) de déshydratation.

Ladite charge comprimée et éventuellement chauffée dans ledit échangeur de type monophasique gaz est ensuite avantageusement introduite dans un four de manière à l'amener à une température d'entrée dans au moins un réacteur adiabatique compatible avec la température de la réaction de déshydratation. Cet échangeur de type monophasique gaz est un échangeur d'une technologie connue de l'Homme du métier qui permet de minimiser les pertes de charge tout en ayant une grande surface d'échange. Cet échange gaz/gaz à basse pression induit une densité de flux de chaleur faible à travers la paroi de l'échangeur (coefficient de transfert faible), ce qui force à avoir une grande surface d'échange. De plus, la perte de pression doit etre minimisée afin de limiter la charge du compresseur de l'étape d). Par exemple cet échangeur peut être un échangeur à plaques pressurisées dans une calandre, de type Packinox fourni par Alphalaval.

## Étape e) de déshydratation

Conformément à l'invention, ladite charge comprimée, et éventuellement chauffée subit une étape e) de déshydratation dans au moins un réacteur adiabatique contenant au moins un lit fixe de catalyseur de déshydratation et dans lequel la réaction de déshydratation a lieu.

Le mélange à l'entrée de l'étape (e) de déshydratation du flux constitué de la charge éthanol et du flux d'eau de dilution comprenant l'éthanol issu de l'étape g) est réalisé de telle manière qu'à l'issue du mélange, le rapport massique eau sur éthanol, appelé taux de dilution, est compris entre 1 et 4. La dilution a pour but d'abaisser les pressions partielles d'éthanol dans le ou les réacteurs et de rendre le procédé plus sélectif en éthylène. Ce rapport massique est ajusté en modifiant le débit d'eau traitée à l'issue de l'étape g) et/ou en modifiant le débit de charge éthanol.

L'étape e) de déshydratation est avantageusement réalisée dans un ou deux réacteurs.

Dans le cas où l'étape e) est mise en oeuvre dans un réacteur adiabatique, ladite charge comprimée, et éventuellement chauffée, est avantageusement introduite dans ledit réacteur à une température d'entrée comprise entre 350 et 550 °C et de préférence entre 400 et 500 °C, et à une pression d'entrée comprise entre 0,3 et 1,8 MPa, et de préférence entre 0,4 et 0,8 MPa.

L'effluent issu dudit réacteur adiabatique de l'étape e) présente avantageusement une température comprise entre 270 et 450 °C et de préférence entre 340 et 430 °C, et une pression de sortie comprise entre 0,2 et 1,6 MPa et de préférence entre 0,3 et 0,8 MPa.

Dans le cas où l'étape e) est mise en oeuvre dans deux réacteurs adiabatiques, ladite charge comprimée, et éventuellement chauffée, est avantageusement introduite dans le premier réacteur à une température d'entrée comprise entre 350 et 550 °C et de préférence à une température comprise entre 370 et 500 °C, et à une pression d'entrée comprise entre 0,3 et 1,8 MPa, et de préférence entre 0,4 et 1,1 MPa.

L'effluent issu du premier réacteur adiabatique sort avantageusement dudit premier réacteur à une température comprise entre 270 et 450 °C et de préférence entre 290 et 390 °C, et à une pression comprise entre 0,3 et 1,7 MPa et de préférence entre 0,3 et 1,0 MPa.

Ledit effluent est ensuite avantageusement introduit dans un four de manière à ce que la température d'entrée dudit effluent dans le deuxième réacteur adiabatique soit comprise entre 350 et 550 °C et de préférence entre 400 et 500 °C. Ledit effluent présente une pression d'entrée dans ledit deuxième réacteur avantageusement comprise entre 0,3 et 1,7 MPa et de préférence entre 0,3 et 0,9 MPa.

L'effluent issu du deuxième réacteur adiabatique sort dudit deuxième réacteur adiabatique à une température avantageusement comprise entre 270 et 450 °C et de préférence entre 340 et 430 °C. La pression de sortie dudit effluent issu du deuxième réacteur adiabatique est avantageusement comprise entre 0,2 et 1,6 MPa et de préférence entre 0,3 et 0,8 MPa.

La température d'entrée du ou des réacteurs peut avantageusement être graduellement augmentée pour éviter la désactivation du catalyseur de déshydratation.

La réaction de déshydratation qui a lieu dans au moins un réacteur adiabatique de l'étape e) du procédé selon l'invention opère avantageusement à une vitesse pondérale horaire comprise entre 0,1 et 20 h$^{-1}$ et de préférence entre 0,5 et 15 h$^{-1}$. La vitesse pondérale horaire est définie comme étant le rapport du débit massique de la charge éthanol pure sur la masse de catalyseur.

Le catalyseur de déshydratation utilisé dans l'étape e) est un catalyseur connu de l'Homme du métier. Ledit catalyseur est de préférence un catalyseur acide amorphe ou un catalyseur acide zéolithique.

Dans le cas où le catalyseur de déshydratation utilisé dans l'étape e) est un catalyseur zéolithique, ledit catalyseur comprend au moins une zéolithe choisie parmi les zéolithes ayant au moins des ouvertures de pores contenant 8, 10 ou 12 atomes d'oxygène (8 MR, 10 MR ou 12 MR). Il est connu en effet de définir la taille des pores des zéolithes par le nombre d'atomes d'oxygène formant la section annulaire des canaux des zéolithes, appelés "member ring" ou MR en anglais. De manière préférée, ledit catalyseur de déshydratation zéolithique comprend au moins une zéolithe présentant un type structural choisi parmi les types structuraux MFI, FAU, MOR, FER, SAPO, TON, CHA, EUO et BEA. De préférence, ledit catalyseur de déshydratation zéolithique comprend une zéolithe de type structural MFI et de manière préférée une zéolithe ZSM-5.

La zéolithe mise en oeuvre dans le catalyseur de déshydratation utilisé dans l'étape e) du procédé selon l'invention peut avantageusement être modifiée par désalumination ou désilication selon toute méthode de désalumination ou désilication connue de l'Homme du métier.

La zéolithe mise en oeuvre dans le catalyseur de déshydratation utilisé dans l'étape e) du procédé selon l'invention ou le catalyseur final peut avantageusement être modifiée par un agent de nature à atténuer son acidité totale et à améliorer ses propriétés de résistance hydrothermales. De préférence, ladite zéolithe ou ledit catalyseur comprend avantageusement du phosphore, de préférence ajouté sous forme $H_3PO_4$ suivi d'un traitement à la vapeur après neutralisation de l'excès d'acide par un précurseur basique tels que par exemple le calcium Ca. De manière préférée, ladite zéolithe comprend une teneur en phosphore comprise entre 1 et 4.5%poids, de préférence entre 1.5 et 3.1%poids poids par rapport à la masse totale du catalyseur.

De préférence, le catalyseur de déshydratation utilisé dans l'étape e) du procédé selon l'invention est le catalyseur décrit dans les demandes de brevet WO/2009/098262 WO/2009/098267, WO/2009/098268, ou WO/2009/098269.

Dans le cas où le catalyseur de déshydratation utilisé dans l'étape e) est un catalyseur acide amorphe, ledit catalyseur comprend au moins un oxyde réfractaire poreux choisi parmi l'alumine, l'alumine activée par un dépôt d'acide minéral et la silice alumine.

Ledit catalyseur de déshydratation amorphe ou zéolithique utilisé dans l'étape e) du procédé selon l'invention peut avantageusement également comprendre au moins une matrice de type oxyde également appelé liant. On entend par matrice selon l'invention, une matrice amorphe, cristallisée, ou comprenant des parties amorphes et cristallisées. Ladite matrice est avantageusement choisie parmi les éléments du groupe formé par les argiles (telles que par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon, utilisés seuls ou en mélange. De préférence, ladite matrice est choisie parmi les éléments du groupe formé par les alumines, les silices et les argiles.

Ledit catalyseur de déshydratation utilisé dans l'étape e) du procédé selon l'invention est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés.

Ledit catalyseur de déshydratation utilisé dans l'étape e) du procédé selon l'invention est avantageusement mis en oeuvre dans au moins un réacteur, en lit fixe ou en lit mobile.

Dans l'étape e) du procédé selon l'invention, les catalyseurs utilisés et les conditions opératoires sont choisis de manière à maximiser la production d'éthylène. Les réactions globales de déshydratation mises en oeuvre dans l'étape e) du procédé selon l'invention sont les suivantes :

$$2\ C_2H_5OH \rightarrow 2\ CH_2{=}CH_2 + 2\ H_2O$$

$$CH_3CH_2OCH_2CH_3 \rightarrow 2\ CH_2{=}CH_2 + H_2O$$

La conversion de la charge éthanol dans l'étape e) est supérieure à 90 %, de préférence 95 % et de manière préférée supérieure à 99 %.

Une conversion inférieure à 90% a pour effet de baisser le rendement global du procédé, une quantité plus importante de DEE non converti en éthylène étant perdue dans les étapes de séparation avale.

La conversion de la charge éthanol est définie, en pourcentage, par la formule suivante :

[1 − (masse horaire d'éthanol en sortie/masse horaire d'éthanol en entrée)]x100.

La masse horaire d'éthanol en entrée et en sortie est mesurée de manière classique par exemple par chromatographie.

L'étape e) dans laquelle la réaction de déshydratation a lieu est avantageusement réalisée dans un ou deux réacteurs. Un réacteur préféré est un réacteur radial fonctionnant en mode ascendant ou descendant. Lors de l'étape e) du procédé selon l'invention, la transformation de la charge s'accompagne de la désactivation du catalyseur de déshydratation par cokage et/ou par adsorption de composés inhibiteurs. Le catalyseur de déshydratation doit donc subir périodiquement une étape de régénération. De préférence, le réacteur est utilisé dans un mode de régénération alterné, aussi appelé réacteur swing, afin d'alterner les phases de réaction et de régénération dudit catalyseur de déshydratation. L'objectif de ce traitement de régénération est de brûler les dépôts organiques ainsi que les espèces contenant de l'azote et du soufre, contenues à la surface et au sein dudit catalyseur de déshydratation. L'étape b) de prétraitement mise en oeuvre dans cette invention permet de réduire la quantité d'impuretés, basiques et organiques, ainsi que les espèces cationiques qui vont venir altérer la durée de cycle du catalyseur. L'élimination de ces espèces permet ainsi de limiter le nombre de régénération du catalyseur.

La régénération du catalyseur de déshydratation utilisé dans ladite étape e) est avantageusement réalisée par oxydation du coke et des composés inhibiteurs sous flux d'air ou sous un mélange air/azote, par exemple en utilisant une recirculation de l'air de combustion avec ou sans eau afin de diluer l'oxygène et maîtriser l'exotherme de régénération. Dans ce cas, on peut avantageusement ajuster la teneur en oxygène en entrée du réacteur par un appoint d'air. La régénération a lieu à pression comprise entre la pression atmosphérique et la pression de réaction.

La température de régénération est avantageusement choisie entre 400 et 600 °C ; elle peut avantageusement varier en cours de régénération. La fin de la régénération est détectée quand il n'y a plus de consommation d'oxygène, signe d'une combustion totale du coke.

L'effluent issu du dernier réacteur adiabatique de l'étape e) est éventuellement envoyé dans un échangeur de type monophasique gaz dans lequel il est "désurchauffé" sans être condensé par échange de chaleur avec la charge comprimée issue de l'étape d), qui elle est surchauffée.

Ledit effluent "désurchauffé" est ensuite avantageusement envoyé dans un deuxième échangeur de type gaz/liquide dans lequel il est condensé partiellement par un échange de chaleur servant à vaporiser la charge de vaporisation.

Ledit effluent est ensuite encore refroidi par échange de chaleur avec la charge éthanol lors de l'étape a) de préchauffe de la charge éthanol.

## Étape f) de séparation

Selon l'invention, l'effluent issu du dernier réacteur adiabatique de l'étape e) subit une étape de séparation f) en un effluent comprenant de l'éthylène à une pression inférieure à 1,6 MPa, préférentiellement inférieure à 0,8 MPa et un effluent comprenant de l'eau.

L'étape f) de séparation dudit effluent issu du dernier réacteur adiabatique de l'étape e) peut avantageusement être mise en oeuvre par toute méthode connue de l'Homme du métier telle que par exemple par une zone de séparation gaz/liquide, et de préférence une colonne de séparation gaz/liquide.

L'effluent comprenant de l'éthylène à une pression inférieure à 1,6 MPa subit ensuite avantageusement une compression. Ladite compression permet de remonter la pression dudit effluent à une pression avantageusement comprise entre 2 et 4 MPa nécessaire à sa purification.

## Étape g) de purification

Selon l'invention, l'effluent comprenant de l'eau issu de l'étape f) de séparation subit une étape g) de purification. L'étape g) de purification peut être mise en oeuvre par toute méthode de purification connue de l'Homme du métier. A titre d'exemple, l'étape g) de purification peut avantageusement être mise en oeuvre par l'utilisation de résines échangeuses d'ions, par ajout d'agents chimiques pour ajuster le pH tels que par exemple la soude ou les amines et par l'ajout d'agents chimiques pour stabiliser les produits, tels que par exemple les inhibiteurs de polymérisation choisis parmi les bisulfites et les tensio-actifs.

Au moins un flux d'eau traitée et au moins un flux de dilution comprenant l'éthanol non converti sont ensuite séparés. La séparation permet d'obtenir un flux d'eau traitée sans éthanol (moins de 10% massique d'éthanol, de préférence moins de 1%), ce qui limite les perte d'éthanol, et peut être mise en oeuvre par toute méthode de séparation connue de l'Homme du métier. A titre d'exemple, la séparation peut avantageusement être mise en oeuvre par distillation, l'utilisation de tamis moléculaires, stripage à la vapeur ou à la chaleur ou par absorption au solvant tels que par exemple les solvants glycolés.

Un flux contenant les gaz légers et l'éthanol, de préférence l'acétaldéhyde et le méthanol, peut avantageusement également être séparé et recyclé vers l'étape f).

Selon l'invention, le flux d'eau de dilution comprenant de l'éthanol issu de l'étape g) de purification est recyclée en amont de l'étape c) de vaporisation.

Le flux d'eau de dilution comprenant de l'éthanol issu de l'étape g) joue le rôle de diluant réactionnel thermique.

### Étape h) de compression

Selon l'invention, le flux comprenant de l'éthylène issu de l'étape f) subit ensuite une compression par des moyens connus de l'Homme du métier : compresseur centrifuge ou volumétrique, éventuellement en plusieurs étages avec refroidissement intermédiaire, les condensats peuvent avantageusement être mélangés à l'alimentation de l'étape g) de purification. Cette compression permet d'amener l'effluent à une pression suffisante pour permettre les éventuelles étapes de purifications qui suivent et dont la pression opératoire est directement dépendante de ce compresseur, aux pertes de charge prêt. De préférence, la pression de décharge est suffisamment élevée pour permettre l'injection directe de l'éthylène dans l'étape i) d'oxydation. Cette pression de décharge se situe entre 1,1 MPa et 5,1 MPa, de préférence entre 1,6 et 3,6 MPa.

Le flux comprimé subit ensuite avantageusement une purification par des moyens connus de l'Homme de métier, par exemple par une ou plusieurs distillations, éventuellement cryogénique, et/ou en utilisant des masses de captation de type résine. La purification du flux comprenant de l'éthylène dépend de sa destination, par exemple de la technologie choisie pour l'étape i) d'oxydation ou de la possibilité ou non de produire de l'éthylène sans l'oxyder (parallèlement a la production d'oxyde d'éthylène).

### Étape i) d'oxydation

Selon l'invention, au moins une partie du flux comprenant de l'éthylène issu de l'étape h) de compression est dirigé vers une étape i) d'oxydation. Cette étape d'oxydation comprend l'ensemble des équipements, catalyseurs et produits chimiques connus de l'Homme du métier permettant une oxydation de l'éthylène en oxyde d'éthylène.

Le catalyseur d'oxydation est avantageusement à base d'argent et la sélectivité de réaction peut avantageusement être optimisée en contrôlant la température de la réaction et, optionnellement, par utilisation d'une dilution au méthane. Ce méthane peut provenir soit d'un stockage, soit d'une unité de production de méthane.

Un ajustement de ces paramètres permet de contrôler la quantité de chaleur dégagée par la réaction, et par conséquent la chaleur extraite via l'évaporation du fluide de trempe et donc la puissance mécanique que la détente et la condensation de ce fluide peut fournir au niveau de l'étape d).

Selon l'invention, la température de la réaction d'oxydation de l'éthylène est contrôlée entre 100 et 500°C, de préférence entre 150 et 300°C grâce à lavaporisation d'un flux appelé "flux de trempe" dans la calandre d'un ou plusieurs réacteur(s) multitubulaire(s), la réaction ayant lieu dans les tubes. Le flux de trempe comprend préférentiellement de l'eau et peut avantageusement être un flux d'eau de procédé, d'eau distillé, d'eau de chaudière ou tout autre flux comprenant de l'eau peu ou pas encrassant. La température de la réaction peut par exemple être contrôlée en faisant varier la pression coté calandre du ou des réacteur(s), ce qui a pour effet de faire varier la température de vaporisation du fluide de trempe.

L'étape i) d'oxydation produit au moins un effluent comprenant de l'oxyde d'éthylène et au moins un flux chaud correspondant au flux de trempe partiellement ou, de préférence, totalement vaporisé qui est ensuite dirigé vers l'étape d) de compression.

Le flux de trempe circule en boucle fermée entre l'étape d) de compression et l'étape i) d'oxydation , il permet d'utiliser la chaleur réactionnelle de la section i) d'oxydation pour compresser la charge de déshydratation.

Usuellement, la vapeur générée par la réaction d'oxydation est envoyée vers un réseau vapeur où elle peut être utilisée, dans la mesure où son niveau thermique le permet, pour subvenir à d'autres besoins en utilités chaudes. La demanderesse a découvert que la quantité et la qualité de la vapeur générée était suffisante pour assurer les besoins en puissance mécanique de l'étape d) de compression, permettant de réaliser une réduction de consommation d'utilités importante et offrant une alternative pertinente, par exemple lorsque les besoins en utilités chaudes sont déjà satisfaits par ailleurs.

### Description des figures

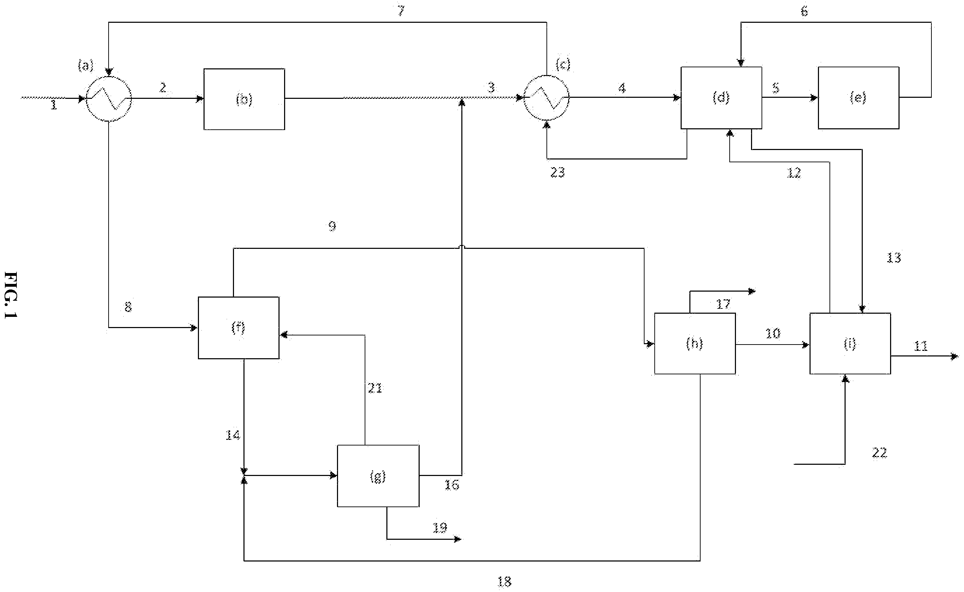

La figure 1 représente schématiquement le procédé de déshydratation de l'éthanol suivi de l'oxydation de l'éthylène produit dans le cas d'une charge éthanol concentrée avec un recyclage d'au moins une partie de l'eau de dilution comprenant de l'éthanol lors de l'étape g) du procédé.

La charge éthanol (1) est préchauffée dans un échangeur a) avec l'effluent de l'étape de deshydratation e) qui pénètre dans l'échangeur via la conduite (7). La charge éthanol préchauffée est ensuite introduite dans une zone de prétraitement b) via la conduite (2). La charge éthanol prétraitée est ensuite mélangée dans la conduite (3) avec le flux d'eau de dilution comprenant de l'éthanol issu de la zone de séparation g) qui est recyclée via la conduites (16) de manière à servir de diluant réactionnel. Ce mélange, constituant la charge de vaporisation, est introduit via la conduite (3) dans l'étape de vaporisation c) dans lequel ledit mélange subit un échange de chaleur avec l'effluent issu de l'étape de déshydratation e) qui pénètre dans l'échangeur via la conduite (23) de manière à produire une charge vaporisée. La chaleur latente, dite également enthalpie de condensation, de l'effluent issu de l'étape de déshydratation e) est utilisée pour vaporiser la charge de vaporisation, sans apport de chaleur externe.

La charge vaporisée est ensuite envoyée via la conduite (4) dans l'étape de compression et surchauffe d). Dans l'étape de compression et surchauffe d), ladite charge vaporisée est comprimée et surchauffée et l'effluent issu, à l'état gazeux, de la section de déshydratation e) est "désurchauffé", sans être condensé. La puissance nécessaire a la compression dans l'étape d) est apportée par le flux (12) constitué du fluide de trempe vaporisé issu de la section i) d'oxydation. Le fluide de trempe condensé est ensuite recyclé vers l'étape i) d'oxydation via la conduite (13).

La dite charge vaporisée, comprimée et surchauffée est ensuite introduite dans l'étape e) dans laquelle elle traverse un four ou tout autre équipement connu de l'Homme de métier de manière à l'amener à une température compatible avec la température de la réaction de déshydratation.

L'effluent issu de la section de déshydratation e) subit ensuite les trois échanges successifs décrits précédemment dans les étapes d), c) et a).

L'effluent issu de l'étape a) est envoyé via la conduite (8) dans la section de séparation f) où il est séparé en un effluent comprenant de l'éthylène (9) et un effluent comprenant de l'eau (14).

L'effluent comprenant de l'éthylène est envoyé dans l'étape h) de compression et purification via la canalisation (9). L'étape h) permet d'amener ledit effluent comprenant de l'éthylène à une pression suffisante pour permettre sa purification et préférentiellement compatible avec la pression d'alimentation de la section i) d'oxydation. L'étape h) permet également de purifier l'effluent comprenant de l'éthylène en séparant au moins un flux comprenant les gaz léger (17) et un flux d'eau et d'éthanol non réagit (18) recyclé vers l'étape g) de purification.

L'effluent compressé et purifié comprenant de l'éthylène (10) issus de l'étape h) est envoyé dans la section i) d'oxydation ou il subit une conversion en oxyde d'éthylène. L'étape i) permet de former un flux comprenant de l'oxyde d'éthylène (11). La chaleur de réaction de l'étape i) permet de vaporiser le fluide de trempe (13) qui est ensuite recyclé via la conduite (12) vers l'étape compression de la charge de déshydratation d).

L'effluent comprenant de l'eau issu de l'étape f) est envoyée via la conduite (14) dans une étape g) de purification. Au moins un flux d'eau de dilution comprenant l'éthanol non converti (16) et au moins un flux d'eau traité sont séparés (19). Un flux contenant les gaz légers et de l'éthanol non converti (21), est également séparé et recyclé vers l'étape f).

La totalité dudit flux d'eau de dilution comprenant l'éthanol non converti issu de l'étape g) de purification est envoyée via la conduite (16) dans l'étape c) de vaporisation.

Les exemples suivants illustrent l'invention sans en limiter la portée.

## Exemples

### Exemple 1 : conforme à l'invention

L'exemple 1 illustre un procédé selon l'invention.

La charge éthanol considérée est produite par fermentation de blé, sans extraction des glutens, par un procédé de type dry milling selon le terme anglo-saxon.

Étape a)

Ladite charge éthanol est introduite, à un débit de 45 664 kg/h dans un échangeur E1 à une pression egale à 1,15 MPa et est chauffée en restant en phase liquide jusqu'à une température de 120 °C contre l'effluent issu du dernier réacteur adiabatique de l'étape e).

Étape b)

La charge éthanol chauffée est prétraitée sur une résine TA801 afin d'éliminer les traces de composés azotés. Lors de ce prétraitement, une partie de l'éthanol est convertie en DEE. Les caractéristiques de la charge éthanol brut et prétraitée sont données dans le Tableau 1.

Tableau 1 : Caractéristiques de la charge éthanol avant et après prétraitement (pourcentages massiques)

|  | CHARGE ETHANOL | ETHANOL APRES PRETRAITEMENT |
|---|---|---|
| ETHANOL | 91,2% | 82,1% |
| H2O | 8,7% | 10.5% |

(suite)

|  | CHARGE ETHANOL | ETHANOL APRES PRETRAITEMENT |
|---|---|---|
| DEE | 0% | 7.3% |
| COMPOSES AZOTE | 0,005% | 0,000% |

Étape c)

La charge de vaporisation, constituée de la charge éthanol prétraitée en mélange avec 140 970 kg/h d'eau traitée et d'éthanol non converti recyclés selon l'étape g) est introduite dans un échangeur E2 à une pression égale à 0,37 MPa. La charge de vaporisation entre dans l'échangeur E2 à 129 °C et est donc déjà vaporisé à 55 % massique. La pression en entrée de l'échangeur E2 a été ajustée de telle manière que l'approche thermique avec le flux issu du dernier réacteur adiabatique de l'étape e) soit au minimum de 15 °C.

Dans l'étape c), la majorité de la chaleur latente de la phase aqueuse de l'effluent issu du dernier réacteur adiabatique de l'étape e) est récupérée pour vaporiser la charge de vaporisation, sans apport de chaleur externe. Ainsi, 51.9 MW sont échangés entre ladite charge de vaporisation et ledit effluent.

Étape d)

La charge vaporisée est ensuite comprimée dans un compresseur radial K1 à multiplicateur intégré de manière à ce que la pression de ladite charge vaporisée soit égale à 0,695 MPa à l'issue de la compression. La puissance mécanique nécessaire a la compression est 8MW. Celle-ci est apportée par une turbine a vapeur à condensation couplée au compresseur prenant à l'aspiration la vapeur apportée par le flux (12) issu de la section d'oxydation i). Ce flux est constitué uniquement de 65914kg/h d'eau de chaudière, sa pression d'entrée dans l'étape h) est 1.5 Mpa à sa température de rosée, ici 200°C. Le flux de décharge de la turbineà condensation est recyclée à 35°C vers l'étape i) d'oxydation.

La charge comprimée, est ensuite chauffée dans un échangeur E3 de type monophasique gaz, grâce à un échange de chaleur avec l'effluent issu du réacteur adiabatique de l'étape e). Dans ledit échangeur de type monophasique gaz, ladite charge comprimée est surchauffée à une température de 404°C et l'effluent issu, à l'état gazeux, du dernier réacteur adiabatique de l'étape e) est "désurchauffé" sans être condensé et présente une température de 234 °C.

Étape e)

L'étape e) de déshydratation comporte deux fours et deux réacteurs adiabatiques en série. Ladite charge comprimée et chauffée dans ledit échangeur de type monophasique gaz est ensuite introduite dans un four de manière à l'amener à une température d'entrée dans le premier réacteur adiabatique de l'étape e) compatible avec la température de la réaction de déshydratation et de conversion du DEE en éthylène hautement endothermique, c'est-à-dire à une température de 470 °C. La température de sortie du dernier réacteur adiabatique de l'étape e) est de 420 °C.

Le piégeage des composés azotés dans l'étape b) de prétraitement permet de réduire significativement la température en entrée du premier réacteur adiabatique de l'étape e).

Ladite charge comprimée et chauffée est introduite dans le premier réacteur adiabatique à une pression d'entrée de 0,595 MPa. La pression de l'effluent en sortie du dernier réacteur adiabatique de l'étape e) est de 0,500 MPa. L'étape e) de déshydratation est opérée à une vitesse pondérale horaire de 7 h$^{-1}$.

Le réacteur adiabatique contient un lit fixe de catalyseur de déshydratation, ledit catalyseur comprenant 80 % poids de zéolithe ZSM-5 traitée avec $H_3PO_4$ de manière à ce que la teneur en phosphore P soit de 3%poids.

La conversion de la charge éthanol dans l'étape e) est de 95 %.

Étape f)

L'effluent issu du dernier réacteur adiabatique de l'étape e) subit ensuite les trois échanges de chaleur précédemment décrits et est envoyé dans une colonne de séparation gaz/liquide. Un effluent comprenant de l'éthylène à une pression égale à 0,36 MPa est séparé ainsi qu'un effluent comprenant de l'eau. Cette séparation est réalisée par l'utilisation d'une colonne de séparation gaz/liquide, avec recyclage de l'eau produite en fond de colonne vers la tête de colonne et après refroidissement et injection d'agent neutralisant.

Étape g)

Un flux d'eau de dilution comprenant l'éthanol non converti (16) ainsi qu'un flux contenant les gaz légers (21) sont ensuite séparés par distillation classique à basse pression de l'eau brute. Un autre flux d'eau traitée est séparé (19) et constitue la purge du procédé, son débit partiel d'eau correspond à l'eau formée par la réaction de déshydratation de l'étape e).

Étape h)

L'effluent comprenant de l'éthylène subit ensuite une compression pour remonter sa pression à 2,78 MPa avant sa purification finale réalisée avec une distillation cryogénique. Un flux de gaz légers est séparé (17) en tête de cette colonne et un flux de condensats comprenant de l'eau et de l'éthanol est recyclé vers l'étape g).

Étape i)

L'effluent d'éthylène purifié issu de l'étape i) est envoyé dans la section j) d'oxydation. Les conditions opératoire de cette section sont les suivantes :

- Conversion par passe : 16%
- Sélectivité : 85%molaire sur l'éthylène
- Taux 02/Éthylène = 7
- Pression d'alimentation du réacteur d'oxydation: 20barg
- Température d'entrée du réacteur 230°C
- Exotherme de la réaction : 40°C

L'exotherme de la réaction, et donc sa sélectivité, est limité grâce à une forte dilution du milieu réactionnel par du méthane qui est recyclé dans le procédé. La purification du méthane est réalisée grâce à une colonne d'absorption utilisant une solution de carbonate de potassium.

Le réacteur est un réacteur multitubulaire permettant de générer 65914 kg/h de la vapeur saturée sous une pression de 1.5 Mpa. Cette vapeur est envoyée dans la section (d) de compression et surchauffe via le flux (12).

La séparation de l'oxyde d'éthylène est réalisée dans une colonne d'absorption a l'eau suivie d'une colonne de stripping.

Un mélange d'oxygène et de méthane d'appoint est injecté dans la section d'oxydation (j) via la conduite (22).

L'oxyde d'éthylène purifié est extrait dans le flux (11) avant d'être envoyé dans une section de conversion en éthylène glycol.

Les différents flux, en kg/h, sont renseignés dans le Tableau 2 :

Tableau 2 : Composition des principaux flux

| Courant | | 1 | 2 | 3 | 4 | 5 | 6 |
|---|---|---|---|---|---|---|---|
| Température | °C | 25 | 120 | 129 | 133 | 404 | 420 |
| Eau | kg/h | 3993 | 3993 | 143730 | 143730 | 143730 | 158602 |
| Ethanol | kg/h | 41671 | 41671 | 39538 | 39538 | 39538 | 2187 |
| DEE | kg/h | | | 3366 | 3366 | 3366 | 14 |
| Ethylène | kg/h | | | | | | 25228 |
| Oxyde d'éthylène | kg/h | | | | | | |
| Autre (leqers+oxygénés) | kg/h | | | | | | 603 |
| Total | | 45664 | 45664 | 186634 | 186634 | 186634 | 186634 |
| | | | | | | | |
| | | | | | | | |
| Courant | | 8 | 19 | 16 | 12 | 13 | 17 |
| Température | °C | 135 | 25 | 143 | 200 | 35 | 25 |
| Eau | kg/h | 158602 | 19680 | 138922 | 65914 | 65914 | |

(suite)

| Courant | | 1 | 2 | 3 | 4 | 5 | 6 |
|---|---|---|---|---|---|---|---|
| Ethanol | kg/h | 2187 | 103 | 2034 | | | 50 |
| DEE | kg/h | 14 | | 14 | | | |
| Ethylène | kg/h | 25228 | | | | | |
| Oxyde d'éthylène | kg/h | | | | | | |
| Autre (legers+oxygénés) | kg/h | 603 | 100 | | | | 503 |
| Total | | 186634 | 19883 | 140970 | 65914 | 65914 | 553 |
| | | | | | | | |
| | | | | | | | |
| Courant | | 9 | 10 | 11 | 22 | | |
| Température | °C | 35 | 25 | 35 | 35 | | |
| Eau | kg/h | 300 | | | | | |
| Ethanol | kg/h | 150 | | | | | |
| DEE | kg/h | 0 | | | | | |
| Ethylène | kg/h | 25228 | 25228 | | | | |
| Oxyde d'éthylène | kg/h | | | 33438 | | | |
| Autre (legers+oxygénés) | kg/h | 503 | | | 8210 | | |
| Total | | 26181 | 25228 | 33438 | 8210 | | |

Les composés C3 et C4 sont des composés hydrocarbonés en C3 et C4.

La sélectivité du procédé de transformation d'éthanol en éthylène est de 99 %.

Elle est calculée de la façon suivante : (Éthylène contenu dans l'effluent comprenant de l'éthylène) / (0.61 *quantité d'éthanol converti) où la quantité d'éthanol converti est l'éthanol contenu dans la charge éthanol avant prétraitement soustrait de l'éthanol contenu dans les flux d'eau purgée et dans l'effluent comprenant de l'éthylène. 0,61 g est la quantité maximale d'éthylène obtenue en déshydratant 1 g d'éthanol pur.

Cet exemple montre que l'utilisation en boucle fermée de la vapeur issue de la section d'oxydation permet d'économiser 8 MW d'énergie mécanique dans l'étape d) de compression.

De plus, la recirculation de l'eau de trempe de la section i) d'oxydation permet d'économiser une étape de recyclage d'eau externe à ce procédé qui permettrait, selon l'art antérieur, de retraiter un débit d'eau équivalent au débit du flux (13). Enfin, la recirculation en boucle fermée permet d'ajuster astucieusement la pression du flux de trempe afin d'optimiser la récupération énergétique dans la turbine de l'étape d).

**Revendications**

1. Procédé de déshydratation d'une charge éthanol en éthylène puis oxydation de l'éthylène en oxyde d'éthylène comprenant :

   a) éventuellement une étape de préchauffe de ladite charge éthanol à une température comprise entre 70 et 130°C par échange de chaleur avec l'effluent issu de l'étape e),
   b) une étape de prétraitement de la charge éthanol sur un solide acide opérant à une température comprise entre 70 et 130°C de manière à produire une charge éthanol prétraitée,
   c) une étape de vaporisation d'une charge de vaporisation comprenant ladite charge éthanol prétraitée et au moins une partie du flux d'eau de dilution comprenant de l'éthanol recyclé à l'issue de l'étape g) dans un échangeur grâce à un échange de chaleur avec l'effluent issu du dernier réacteur de l'étape e), ladite charge de vaporisation étant introduite dans ladite étape de vaporisation à une pression comprise entre 0,1 et 1,4 MPa, choisie la plus haute possible, de manière à ce que la différence entre la température de condensation de l'effluent de l'étape e) de déshydratation et la température de vaporisation de ladite charge de vaporisation soit supérieure ou égale à 2 °C, de manière à produire une charge vaporisée,

d) une étape de compression et surchauffe de ladite charge vaporisée dans un compresseur de manière à produire une charge comprimée et à la température d'alimentation de la section de déshydratation e), ledit compresseur étant entraîné par une turbine à condensation prenant à l'aspiration le flux de trempe vaporisé dans la section i) d'oxydation, le flux de décharge sortant de la turbine à condensation étant recyclé vers l'étape i) d'oxydation,

e) une étape de déshydratation de la charge comprimée, ladite charge présentant un rapport massique eau sur éthanol compris entre 1 et 4, dans au moins un réacteur adiabatique contenant au moins un catalyseur de déshydratation et dans lequel la réaction de déshydratation a lieu, opérant à une température d'entrée comprise entre 350 et 550 °C et à une pression d'entrée comprise entre 0,3 et 1,8 MPa,

f) une étape de séparation de l'effluent issu du dernier réacteur adiabatique de l'étape e) en un effluent comprenant de l'éthylène à une pression inférieure à 1,6 MPa et un effluent comprenant de l'eau,

g) une étape de purification d'au moins une partie de l'effluent comprenant de l'eau issu de l'étape f) et la séparation en au moins un flux d'eau traitée et un flux d'eau de dilution comprenant de l'éthanol, ce dernier étant recyclé en amont de l'étape c) de vaporisation,

h) une étape de compression de l'effluent comprenant de l'éthylène issu de l'étape f),

i) une étape d'oxydation de l'effluent issu de l'étape h) en oxyde d'éthylène, cette étape d'oxydation comprenant au moins un réacteur tubulaire d'oxydation refroidi par vaporisation dudit flux de trempe issu de l'étape d), ledit flux trempe ainsi réchauffé étant recyclé vers l'étape d).

**2.** Procédé selon la revendication 1 dans lequel ladite charge comprimée est chauffée dans un échangeur de type monophasique gaz, grâce à un échange de chaleur avec l'effluent issu du dernier réacteur adiabatique de l'étape e).

**3.** Procédé selon l'une des revendications 1 à 2 dans lequel le flux comprimé à l'issue de ladite étape h) subit une purification.

**4.** Procédé selon l'une des revendications 1 à 3 dans lequel la pression de la charge comprimée est comprise entre 0,3 et 1,8 MPa.

**5.** Procédé selon l'une des revendications 1 à 4 dans lequel l'effluent issu du dernier réacteur adiabatique de l'étape e) présente en sortie du dernier réacteur adiabatique de l'étape e) une température comprise entre 270 et 450°C, et une pression comprise entre 0,2 et 1,6 MPa.

**6.** Procédé selon l'une des revendications 1 à 5 dans lequel l'étape e) de déshydratation est réalisée dans un ou deux réacteurs.

**7.** Procédé selon l'une des revendications 1 à 6 dans lequel ledit catalyseur de déshydratation utilisé dans l'étape e) est un catalyseur acide amorphe ou un catalyseur acide zéolithique.

**8.** Procédé selon l'une des revendications 1 à 7 dans lequel ladite charge éthanol comprend un pourcentage massique en éthanol compris entre 35 et 99,9% poids.

**9.** Procédé selon l'une des revendications 1 à 8 dans lequel l'étape b) éventuelle de prétraitement est complétée par un prétraitement utilisant une résine échangeuse d'anion.

**Patentansprüche**

**1.** Verfahren zur Dehydrierung einer Ethanolcharge zu Ethylen, dann Oxidierung des Ethylens zu Ethylenoxid, umfassend:

a) eventuell einen Schritt der Vorerhitzung der Ethanolcharge bei einer Temperatur zwischen 70 und 130°C durch Wärmeaustausch mit dem aus dem Schritt e) stammenden Abfluss,

b) einen Schritt der Vorbehandlung der Ethanolcharge auf einem sauren Feststoff, der bei einer Temperatur zwischen 70 und 130 °C wirkt, um eine vorbehandelte Ethanolcharge zu erzeugen,

c) einen Schritt der Verdampfung einer Verdampfungscharge, umfassend die vorbehandelte Ethanolcharge und mindestens einen Teil des Verdünnungswasserstroms, umfassend Ethanol, das nach dem Schritt g) in einem Wärmetauscher durch einen Wärmeaustausch mit dem aus dem letzten Reaktor des Schrittes e) stammenden Abfluss rezykliert wurde, wobei die Verdampfungscharge in den Verdampfungsschritt mit einem Druck zwischen 0,1 und 1,4 MPa eingeleitet wird, welcher möglichst hoch gewählt wird, so dass der Unterschied zwischen der Kondensationstemperatur des Abflusses aus dem Schritt e) der Dehydrierung und der Verdampfungstemperatur der Verdampfungscharge größer oder gleich 2 °C ist, um eine verdampfte Charge zu erzeugen,

d) einen Schritt der Kompression und Überhitzung der verdampften Charge in einem Kompressor, um eine komprimierte und bei der Versorgungstemperatur des Dehydrierungsabschnitts e) liegende Charge zu erzeugen, und bei der Versorgungstemperatur des Dehydrierungsabschnitts e), wobei der Kompressor von einer Kondensationsturbine angetrieben wird, die an der Ansaugung den in dem Oxidierungsabschnitt i) verdampften Gasstrom heranzieht, wobei der Entladestrom, der aus der Kondensationsturbine austritt, zum Oxidierungsschritt i) hin rezykliert wird,

e) einen Schritt der Dehydrierung der komprimierten Charge, wobei die Charge ein Massenverhältnis Wasser zu Ethanol zwischen 1 und 4 aufweist, in mindestens einem adiabatischen Reaktor, der mindestens einen Dehydrierungskatalysator enthält, und in dem die Dehydrierungsreaktion stattfindet, der bei einer Eingangstemperatur zwischen 350 und 550 °C und bei einem Eingangsdruck zwischen 0,3 und 1,8 MPa wirkt,

f) einen Schritt der Trennung des aus dem letzten adiabatischen Reaktor des Schrittes e) stammenden Abflusses in einen Abfluss, umfassend Ethylen mit einem Druck unter 1,6 MPa, und einen Abfluss, umfassend Wasser,

g) einen Schritt der Reinigung mindestens eines Teils des Abflusses, umfassend Wasser aus dem Schritt f), und Trennung in mindestens einen behandelten Wasserstrom und einen Verdünnungswasserstrom, umfassend Ethanol, wobei dieser letztgenannte stromaufwärts zu dem Verdampfungsschritt c) rezykliert wird,

h) einen Schritt der Kompression des Abflusses, umfassend Ethylen aus dem Schritt f),

i) einen Schritt des Oxidierens des aus dem Schritt h) stammenden Abflusses zu Ethylenoxid, wobei dieser Oxidierungsschritt mindestens einen Oxidierungs-Rohrreaktor umfasst, der durch Verdampfung des aus dem Schritt d) stammenden Gasstroms gekühlt wird, wobei der so wieder erhitzte Gasstrom zum Schritt d) hin rezykliert wird.

2. Verfahren nach Anspruch 1, bei dem die komprimierte Charge in einem einphasigen Gastauscher mit einem Wärmeaustausch mit dem aus dem letzten adiabatischen Reaktor des Schrittes e) stammenden Abfluss erhitzt wird.

3. Verfahren nach einem der Ansprüche 1 bis 2, bei dem der nach dem Schritt h) komprimierte Strom einer Reinigung unterzogen wird.

4. Verfahren nach einem der Ansprüche 1 bis 3, bei dem der Druck der komprimierten Charge zwischen 0,3 und 1,8 MPa beträgt.

5. Verfahren nach einem der Ansprüche 1 bis 4, bei dem der aus dem letzten adiabatischen Reaktor des Schrittes e) stammende Abfluss am Ausgang des letzten adiabatischen Reaktors des Schrittes e) eine Temperatur zwischen 270 und 450 °C und einen Druck zwischen 0,2 und 1,6 MPa aufweist.

6. Verfahren nach einem der Ansprüche 1 bis 5, bei dem der Dehydrierungsschritt e) in einem oder zwei Reaktoren durchgeführt wird.

7. Verfahren nach einem der Ansprüche 1 bis 6, bei dem der in dem Schritt e) verwendete Dehydrierungskatalysator ein amorpher saurer Katalysator oder in zeolithischer saurer Katalysator ist.

8. Verfahren nach einem der Ansprüche 1 bis 7, bei dem die Ethanolcharge einen Massenprozentsatz an Ethanol zwischen 35 und 99,9 Gew.-% umfasst.

9. Verfahren nach einem der Ansprüche 1 bis 8, bei dem der mögliche Vorbehandlungsschritt b) durch eine Vorbehandlung ergänzt wird, die ein Anionentauschharz verwendet.

## Claims

1. A process for the dehydration of an ethanol feed to ethylene then oxidation of the ethylene to ethylene oxide, comprising:

a) an optional step for preheating said ethanol feed to a temperature in the range 70°C to 130°C by exchange of heat with the effluent obtained from step e);

b) a step for pre-treatment of the ethanol feed over an acidic solid operating at a temperature in the range 70°C to 130°C in order to produce a pre-treated ethanol feed;

c) a step for vaporization of a vaporization feed comprising said pre-treated ethanol feed and at least a portion of the flow of diluting water comprising ethanol recycled to the outlet from step g) in an exchanger by means of an exchange of heat with the effluent obtained from the last reactor of step e), said vaporization feed being introduced into said vaporization step at a pressure in the range 0.1 to 1.4 MPa, chosen as high as possible so that the difference between the condensation temperature of the effluent from dehydration step e) and the vaporization temperature of said vaporization feed is greater than or equal to 2°C, so as to produce a vaporized feed;

d) a step for compressing and superheating said vaporized feed in a compressor so as to produce a feed which is compressed and at the temperature for supplying to the dehydration section e), said compressor being driven by a condensing turbine the intake for which is the quench flow vaporized in the oxidation section i), the exhaust flow leaving the condensing turbine being recycled to the oxidation step i);

e) a step for dehydration of the compressed feed, said feed having a ratio of water to ethanol in the range 1 to 4 by weight, in at least one adiabatic reactor containing at least one dehydration catalyst and in which the dehydration reaction takes place, operating at an inlet temperature in the range 350°C to 550°C and at an inlet pressure in the range 0.3 to 1.8 MPa;

f) a step for separating the effluent obtained from the last adiabatic reactor of step e) into an effluent comprising ethylene at a pressure of less than 1.6 MPa and an effluent comprising water;

g) a step for purification of at least a portion of the effluent comprising water obtained from step f) and separation into at least a flow of treated water and a flow of diluting water comprising ethanol, the latter being recycled upstream of the vaporization step c);

h) a step for compression of the effluent comprising ethylene obtained from step f);

i) a step for oxidation of the effluent obtained from step h) into ethylene oxide, this oxidation step comprising at least one tubular oxidation reactor cooled by vaporization of said quench flow obtained from step d), said quench flow which has thus been reheated being recycled to step d).

2. The process according to claim 1, in which said compressed feed is heated in a single phase type gas exchanger by means of an exchange of heat with the effluent obtained from the last adiabatic reactor of step e).

3. The process according to claim 1 or claim 2, in which the compressed flow obtained from said step h) undergoes a purification.

4. The process according to one of claims 1 to 3, in which the pressure of the compressed feed is in the range 0.3 to 1.8 MPa.

5. The process according to one of claims 1 to 4, in which the effluent obtained from the last adiabatic reactor of step e) is at a temperature in the range 270°C to 450°C, and at a pressure in the range 0.2 to 1.6 MPa.

6. The process according to one of claims 1 to 5, in which the dehydration step e) is carried out in one or two reactors.

7. The process according to one of claims 1 to 6, in which said dehydration catalyst used in step e) is an amorphous acid catalyst or a zeolitic acid catalyst.

8. The process according to one of claims 1 to 7, in which said ethanol feed comprises a percentage by weight of ethanol in the range 35% to 99.9% by weight.

9. The process according to one of claims 1 to 8, in which the optional step b) for pre-treatment is supplemented by a pre-treatment using an anion exchange resin.

FIG. 1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4232179 A
- WO 2007134415 A
- US 4396789 A
- WO 2011002699 A
- US 3119837 A
- EP 0496470 B1
- WO 2009098262 A
- WO 2009098267 A
- WO 2009098268 A
- WO 2009098269 A

**Littérature non-brevet citée dans la description**

- **H. KNÖZINGER ; R. KÖHNE.** The Deshydration of Alcohols over Alumina. I:The reaction scheme. *Journal of Catalysis,* 1966, vol. 5, 264-270
- **S.N. CHAUDHURI.** Reactions of ethanol over ZSM-5. *Journal of Molecular Catalysis,* 1990, vol. 62
- **MIKE BRUSCINO.** Scientific Design's Ethanol to Monoethylene Glycol Technology. *Hydrocarbon World,* 2010, vol. 5 (2), 15-17